# EUROPEAN PATENT APPLICATION

(11) **EP 0 771 560 A1**
(43) Date of publication of application: **07.05.1997**
(21) Application number: 95500122.7
(22) Date of filing: 25.08.1995
(51) Int. Cl.: A61K 31/00, A61K 31/44

(54) **Use of 2- N-(1,2-benzoisothiazolyl-3(2H)one-1,1-dioxide ethyl 5-ethoxycarbonyl-2,4,6-trimethyl-1,4-dihydropiridine-3-carboxylate in the treatment of vascular disorders**

(71) Applicant: LABORATORIOS ALTER, S.A., 28036 Madrid (ES)
(72) Inventor: Sunkel Letelier, Carlos, E-28033 Madrid (ES); Cillero Corral, Fco. Javier, E-28003 Madrid (ES); Fau de Casa-Juana Munoz, Miguel, E-28043 Madrid (ES); Ortega Navarrete, Pilar, E-28036 Madrid (ES); Priego Fdez. del Campo, Jaime, E-28030 Madrid (ES); Santos Gill, Luis, E-28006 Madrid (ES)
(74) Representative: Ungria Lopez, Javier

(57) **Abstract**

It mainly comprises the application of this product as a therapeutic agent used to treat patients with arteriosclerosis and/or re-stenosis that suffer a serious risk of occlusion of the vessels affected by the disease.

Said product can be administered orally or parenterally, combined with other drugs or alone, to patients with arteriosclerotic disease to reduce the atheromatous lesion as well as to prevent re-stenosis of the blood vessels.

## Description

The present invention fits in the field of drugs capable of having an influence on the regulation of arteriosclerotic disease and of re-stenosis.

More specifically, the present invention refers to new applications of 2-[N-(1,2-benzoisothiazolyl-3(2H)one-1,1-dioxide] ethyl 5-ethoxycarbonyl-2,4,6-trimethyl-1,4-dihydropyridine-3-carboxylate, with the code name PCA-4230, as a drug with an anti-arteriosclerotic and antiproliferous effect on human artery muscle cells.

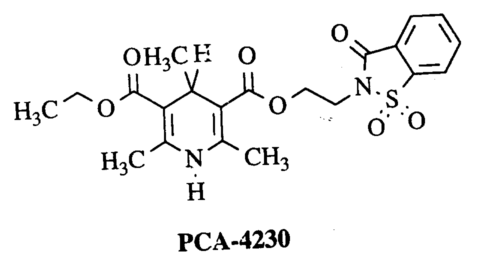

PCA-4230 has been described for the first time in German patent no. DE-A-36 79 76, as an antithrombotic drug.

### PRIOR ART OF THE INVENTION

The key processes responsible for the formation of the plaque in arteriosclerosis are: the accumulation of cholesterol, caused by the increase in the net captation of atherogenic lipoproteins; recruitment and cell proliferation, caused by chemotactic and mitogenic activities; the expansion of the extracellular matrix and the accumulation of collagen, due to the increase of synthetic activity of the cells of the intima and to the high cell death rates caused by the increase of the metabolic needs in an unfavourable medium. In turn, cell death is related to neo-vascularization, fibrosis and calcareous degeneration with the subsequent complications of breakage of the plaque hemorrhagic thrombosis. (For a review: R. Ross, Nature (1993), 362:801-809).

The gap junctions between the vascular smooth cells seem to regulate the cell communication and to be one of the critical factors in the development of arteriosclerosis. These gaps are intramembranous proteins. Six proteins form a circular channel that can open or close, through which molecules up to 1200 daltonsmay be transferred from one cell to another. There is experimental evidence of the participation of proliferation processes, by cell communication in the development of arteriosclerosis. Likewise, it is known that intracellular transfer of molecules is increased in muscle cells derived from arteriosclerotic aorta plaques in comparison with cells of normal aortal tissue. Thus, a lesion produced in the iliac artery causes the same pattern of cell proliferation in the entire aorta, while transfusions of animal serum with arterial lesions to healthy animals did not cause, in the latter, smooth muscle cell proliferation (M.A. Reidy, Arteriosclerosis (1990), 10:298-305).

Another functional characteristic of arteriosclerosis, even in pre-stenotic stages, are severe vasomotor abnormalities caused in part by endothelial dysfunction, that is characterized by incapacity of the endothelial cells of the vessel to synthesize vasodilator substances, such as Prostacycline (PGI₂) and nitric oxide (NO,EDRF) (M.L. Armstrong, Am. J. Hypertens. (1991), 7:503S-511S).

The regression of arteriosclerosis implies the interruption of these pathological processes and the existence of a change in the evolution of the same.

Over the last few years, the effects that different drugs have on the evolution of the disease, have been studied with not very conclusive results.

Ca²⁺ antagonists can block specific stages of arteriogenesis. Thus, Ca²⁺ channel blockers reduce the endothelial damage and the permeability of the intima, block cell proliferation in the intima, inhibit the accumulation of lipids by different mechanisms and reduce the exacerbated vasoconstrictor response (A.M. Keogh, Am. J. Hypertens. (1991), 4:512S-518S).

Apparently, Ca²⁺ antagonists should cause the regression of the atheromatous lesions and their effect should be greater than the one obtained with the reduction of the risk factors. However, the results obtained up to now in different clinical studies are contradictory. Ca²⁺ antagonists seem to reduce only the progression of minimum heart lesions. It is possible that the next publication of the results of the clinical study MIDAS (Multicenter Isradipine/Diuretic

Atherosclerosis Study, Blood Press. (1994),3 (suppl 1):29-35) contributes to clarifying the possible anti-atherogenic activity of this group of drugs.

However, in the acute stages of arterial arteriosclerotic disease, treatment with platelet function inhibitors, anticoagulants and beta-blocking drugs is also required to prevent complications that can mean a risk for the patient's life. Therefore, an optimal strategy for secondary prevention of coronary arterial disease, should be directed towards the critical cell and molecular mechanisms in the beginning, progression or stabilization of the disease.

Due to the importance of thrombosis in the pathogensis and progression of ateromatous lesions, it is necessary to develop intervention strategies directed towards thrombo-resistance of the endothelial cell and to thrombo-regulation. However, the results of clinical studies such as the one carried out with a new antiplatelet drug, stable analogue of prostacycline, active orally (TRK 100), in patients with atheromatous lesions of the carotoid artery, show that although the compound significantly reduced platelet aggregation and the plate accumulation rate in the lesion, there were no significant reductions of the plaque compared to basal values. The data obtained suggest that short-term therapy with an antiplatelet drug, TRK-100 in this case, has an inhibitory effect on the accumulation of platelets in the atheroma, but does not cause significant changes in the size of the atheromatous plaque (Y. Isaka, Thromb. Haemost. (1991), 65:344-350).

This lack of activity of the antiplatelet compounds on the regression of the arteriosclerotic disease, has been described for other compounds with this activity, such as Ketanserin (Ketaserin Trial Group, Br. Med. J., (1989),298: 424-430); Thiclopydine (Janzon, I., Bergquist, D., Boderg, J. et al., J. Intern. Med., (1990), 227:301-308) or Picotamide (Balsano, F., Violi, F. and ADEP Group, Circulation, (1993), 87:1563-1569). In the clinical studies carried out with these drugs, it has been shown that all of them, except Ketanserin, reduce the incidence of myocardial infarction, ictus, and other cardiovascular complications, due to the activation of platelets and which are frequent in arteriosclerotic disease. However, said drugs do not modify the evolution itself of the lesion that causes said pathologies (Verstreatem M., Br. Med. Bull. (1994), 50:946-965).

The compound object of this patent, 2-[N-(1,2-benzoisothiazolyl-3(2H)one-1,1-dioxide]ethyl 5-ethoxycarbonyl-2,4,6-trimethyl-1,4-dihydropyridine-3-carboxylate has been previously described and its use patented as a drug with antiplatelet activity. Said drug has an original action mechanism related to the inhibition of Ca²⁺ entry into platelets through channels operated by receptors and with selective inhibition of phosphodiesterase V, specific of cyclic GMP (M.T. Alonso, C. Villalobos and A. Sánchez, Biochim. Biophys. Acta (1992), 1104:257-260; M. Lombardía, R.E. Catalán et al. Cell. Pharmacol. sent for publication Surprisingly, said compound, aside from its antiplatelet activity, in accordance with the claims of patent DE-A-36 79 76 has recently demonstrated, having antiproliferous and hypolipidemic activity in studies on vascular smooth muscle cells and in clinic tests carried out on patients.

This effect of the compound is not related to its antiplatelet activity, as it is shown in examples included hereinafter.

Along these lines, the present invention surprisingly provides new applications of 2-[N-(1,2-benzoisothiazolyl-3(2H)one-1,1-dioxide]ethyl 5-ethoxycarbonyl-2,4,6-trimethyl-1,4-dihydropyridine-3-carboxylate.

### BRIEF DESCRIPTION OF THE FIGURES

Figure 1a is a bar diagram wherein the effect of PCA-4230 on DNA synthesis induced by BFS in A10 cells is represented.

Figure 1b is a bar diagram wherein the effect of PCA-4230, ASA and Thiclopydine on the proliferation of A10 cells is represented.

Figure 2a is a bar diagram wherein the number of coupled cells in cultures by explant of normal and arteriosclerotic arteries (human aorta smooth muscle cells) is represented.

Figure 2b is a bar diagram wherein the number of coupled cells in ateromatous cell cultures and in the same type of cells treated with 200 ng/ml of PCA-4230.

### DETAILED DESCRIPTION OF THE INVENTION

The present invention refers to new applications of 2-[N-(1,2-benzoisothiazolyl-3(2H)one-1,1-dioxide]ethyl 5-ethoxycarbonyl-2,4,6-trimethyl-1,4-dihydropyridine-3-carboxylate.

The studies carried out, set forth in the Examples hereinafter, have surprisingly shown that PCA-4230 has properties useful for the prevention and treatment of diseases such as arteriosclerosis, either primarily or as a complication of other types of pathological situations and/or incipient re-stenosis subsequent to angioplasty. In this way, PCA-4230 could modify the course of the disease, improving the quality of life of these patients.

The invention also concerns the use of PCA-4230 and of a pharmaceutically acceptable preparation for the manufacture of a drug useful in the treatment of arteriosclerosis in the event that this is the primary disease as well as when it is a complication in patients affected by other types of pathologies.

Scientific experimentation with PCA-4230 has been carried out in "in vitro" studies with a rat smooth muscle cell line (A10, American Type Tissue Collection) in which the capacity of PCA-4230 to inhibit cell proliferation induced by bovine fetal serum (BFS) has been studied. Likewise, we have demonstrated that this inhibition of proliferation is due to a suppressive effect of the drug on the transcription of the c-fos and c-jun proto-oncogenes. A specific effect that is only manifested in cells capable of replication in response to mitogenic stimuli and that, obviously, is not related to the activity of the compound on the platelets, that are particles lacking DNA and, therefore, the capacity to proliferate.

Another series of experiments were carried out on healthy human artery smooth muscle cells and ones coming from atheromatous plaques. In these cells, the capacity of intracellular communication of both types of cells and the effect of PCA-4230 on this cell communication process have been measured. The results of these experiments showed that PCA-4230 is capable of returning intracellular communication of human arteriosclerotic cells to normal, making these cells act as healthy ones.

Therefore, in accordance with the above, the present invention refers to new and surprising applications of PCA-4230, that can be summarized in the following points:
(1) Application as a therapeutic agent to treat pathological situations that require the inhibition of the proliferation of smooth muscle cells, such as restenosis subsequent to angioplasty or to other clinical situations and arteriosclerosis.
(2) Application as a therapeutic agent of vascular disease with anti-atherogenic effects.
(3) Application for the treatment of patients with intermittent claudication who suffer the risk of developing arterial obstruction.
(4) Application as a coadjuvant in the treatment of arteriosclerotic complications caused by the existence of other pathological states that favor the development of arteriosclerosis.

Likewise, PCA-4230 as such or in the form of a pharmaceutically acceptable preparation can be used in the manufacture of drugs for the treatment of patients with vascular disease, including different pathologies, such as arteriosclerosis and ischemic and chronic occlusive disorders.

The present compound is formulated for its use as a pharmaceutical product, also comprising pharmaceutically acceptable vehicles or solvents. The compositions may be prepared by following conventional methods and may be administered in any pharmaceutically suitable manner.

For example, the solid forms to be administered orally can contain, aside from the active ingredient, solvents such as lactose, dextrose, saccharose, cellulose, cornstarch or potato starch; lubricants such as silica, talc, stearic acid and/or polyethyleneglycols; agglutinants such as starches, arabic gum, gelatin, methylcellulose, carboxymethylcellulose or polyvinylpyrrolidone; disintegrating agents such as starch, alginic acid, alginates or starch sodium glycolate; effesvescent mixtures; authorized dyes, sweeteners; wetting agents such as lecithin, polysorbates or lauryl sulfates.

Such mixtures can be prepared by known processes, such as for example, processes to prepare mixtures, granulated preparations, tablets, pills, dragees or capsules. They can also be prepared by processes to coat the previous ones, such as for example pills or capsules with an enteric coating.

Liquid dispersions to be administered orally can be syrups, emulsions or suspensions. Aside from the active ingredient, the syprups can contain saccharose, or saccharose with glycerol and/or mannitol and/or sorbitol. In particular, a syrup for diabetic patients can contain, aside from the active ingredient, only products that are not metabolized to glucose or that only do so in a small proportion, such as sorbitol for example. As a vehicle, the suspensions and emulsions can contain, for example, a natural rubber, agar, sodium alginate, pectin, methylcellulose, carboxymethylcellulose or polyvinyl alcohol.

Another acceptable way to administer the drug is as a suppository.

The suspensions or solutions to be injected intramuscularly can contain, along with the active compound, a pharmaceutically acceptable vehicle, such as for example, apyrogenic distilled water, olive oil, ethyl oleate, glycols such as propylene glycol and, if desired, a suitable amount of lidocaine hydrochloride. The solutions for intravenous injection or infusion can contain a vehicle, for example, apyrogenic sterile water, that is known as water for injections. However, these preparations may also be sterile isotonic saline solutions. Alternatively, the present compound can be microencapsulated using liposomes.

Besides, PCA-4230 can be used together with one or more agents with vascular activity, such as β-blocking agents, calcium channel antagonists and other drugs with vasodilating activity, as well as hypolipidemic drugs.

PCA-4230 can be administered orally, combined with other drugs or alone, to patients affected with chronic obstructive vascular disease for the purpose of reducing stenosis of the vessel and the rates of risk of arterial obstruction.

### EMBODIMENTS OF THE INVENTION

The present invention is based on the Examples given hereinafter, wherein different studies carried out to manifest the new applications of PCA-4230 are shown. These examples are simply illustrative of the invention and by no means do they limit its scope, defined exclusively by the attached claim set.

### EXAMPLE 1

### Inhibition produced by PCA-4230 on smooth muscle cell proliferation induced by bovine fetal serum

Materials and Methods. The comparative study on smooth muscle cell proliferation of PCA-4230 and other compounds structurally related to it (compounds according to examples 2 and 5 described in German patent DR-A 36 79 76) on smooth muscle cell proliferation, was carried out. Likewise, another series of experiments wherein its antiproliferous activity was compared with that of two drugs with recognized antiplatelet activity such as aspirin and Thiclopydine was done (Pantrano, C., N. Engl. Med. J. (1994), 330:1287-1294; Editorial, The Lancet (1991); 459-460).

In these experiments a rat aorta smooth muscle cell line obtained from American Type Culture Collection (ATCC;A10 CRL 1476) was used. The cells were grown in a humidified atmosphere (37^{º} C; 95% air - 5% CO₂) in Dulbecco's Modified Eagle Medium (DMEM) containing 10 % bovine fetal serum supplemented with 20 mM L-glutamine, 100 IU/ml penicillin, 100 µg/ml streptomycin and 0.25 µg/ml Amphotericin B (antibiotic-antimycotic solution, Gibco). The medium was replaced every two days.

To study the effect of PCA-4230 and of the other compounds, the DNA synthesis was measured by deterimining the inclusion of BrdU (an analogue of thymidine). The cells were isolated by trypsinization (trypsin 0.05% in EDTA 0.02%) and were seeded on glass cover-glasses in culture plates of 24 cups, at a density of 10⁴ cells per cup. The cells were grown for 24 hours in DMEM with 10% BFS to allow adhesion of the cells to the slides. Afterwards, the cells were washed with serum-free DMEM containing 0.2% bovine serum albumin (BSA). They were incubated for 48 hours in the same medium to obtain quiescent cells. Afterwards, proliferation was induced by replacement of the medium by DMEM containing 10% bovine fetal serum, along with the vehicle or drugs at the suitable concentrations (0.5 to 50 µM for PCA-4230 and 50 µM for the rest of the compounds): The cells were grown in these conditions for 16 hours and, afterwards, BrdU was added and the culture was continued for two to three hours.

The inclusion of BrdU is displayed by immunocytochemical staining of the nuclei with a specific anti-BrdU monoclonic antibody.

The expression of the c-fos and c-jun proto-oncogenes was analyzed in cells fasted for 48h (cultured in serum-free DMEM, containing BSA 0.2%) and subsequently stimulated for 30 minutes with 10% FCS in the presence of absence of PCA-4230 (5x10⁻⁵M). The total RNA was extracted from said cells using a previously described method (P Chomczynski and N. Sacchi (1987), Ann. Biochem. 162: 156-159).

Immediately, the extracted RNA (10 µg) was resolved by eletrophoresis in agarose gel (1.2% agarose + formaldehyde + formamide). The gel was then transferred to a nylon membrane and this was hybridized with specific probes for c-fos and c-jun (previously labeled with [³²P]-deoxy CTP). The membrane was finally exposed to a sensitive film (Kodak X-OMAT) for 8-17 hours at -70^{º} C. The level of expression of the oncogenes was brought back to normal by using a specific probe for β-actin.

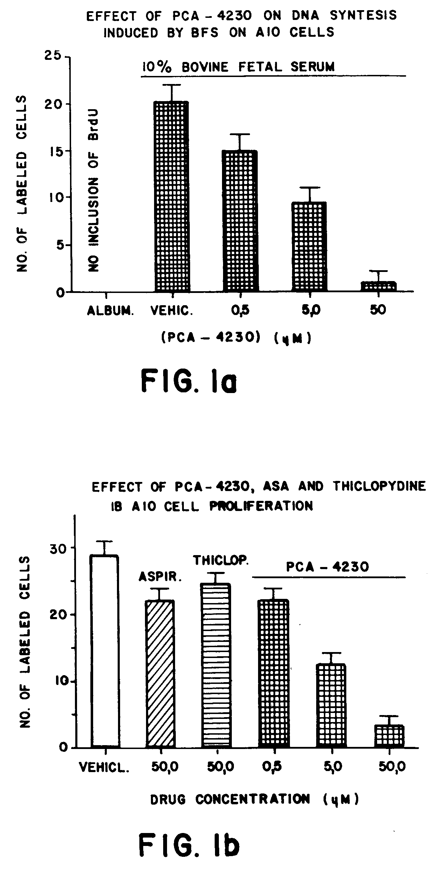

Results. The results obtained are described in Tables Ia and Ib and in Figures 1a and 1b. The percentages of cells that showed the inclusion of BrdU in the control group as well as in the ones treated with each one of the concentrations of PCA-4230 studied, the vehicle, the compounds structurally related to PCA-4230 (Table Ia and Fig 1a) and with the chosen antiplatelet drugs (Table Ib and Fig. 1b) are expressed.

Incubation with bovine fetal serum 10% induced stimulation of cell proliferation and the subsequent inclusion of BrdU in the DNA of the cultured cells, while incubation with bovine albumin in the same proportion did not induce the inclusion of BrdU at all, which shows that the cultures used in these experiments were quiescent and synchronized. Under these conditions, PCA-4230 inhibited cell proliferation induced by bovine fetal serum proportionally to the concentration of PCA-4230 used. The structural homologues of PCA-4230 according to examples 2 and 5 of German patent DE-A-36 79 76 did not modify the inclusion of BrdU in the cultured cells, when they were tested in the same conditions at the conclentration of 50 µM.

**TABLE Ia**

| The data represent the average ± S.D. of three experiments in triplicate | | | |
|---|---|---|---|
| COMPOUND | CONCENTRATION (µM/L) | % LABELED CELLS (Average ± S.D.) | % INHIBITIÓN |
| VEHICLE | ---- | 20,3 ± 1,7 | 0,00 |
| PCA-4230 | 50,0 | 1,0 ± 0,6 | 95,1 |
| | 5,0 | 9,5 ± 1,1 | 53,2 |
| | 0,5 | 15,0 ± 2,3 | 26,1 |
| COMPOUND 2 | 50,0 | 21,2 ± 1,8 | 0,0 |
| COMPOUND 5 | 50,0 | 19,8 ± 2,4 | 2,5 |

**TABLE Ib**

| The data represent the average ± S.D. of two experiments in triplicate | | | |
|---|---|---|---|
| COMPOUND | CONCENTRATION (µM/L) | % LABELED CELLS (Average ± S.D.) | % INHIBITIÓN |
| VEHICLE | ---- | 29,8 ± 2,1 | 0,0 |
| PCA-4230 | 50,0 | 3,6 ± 0,3 | 87,9 |
| | 5,0 | 12,7 ± 1,4 | 57,4 |
| | 0,5 | 22,3 ± 0,9 | 25,0 |
| THICLOPYD. | 50,0 | 24,7 ± 1,5 | 17,1 |
| ASPIRIN | 50,0 | 22.2 ± 1,8 | 25,5 |

Likewise, the two strong antiplatelet drugs studied (aspirin and Thiclopydine) showed, at the tested concentrations, a weak inhibitory effect of cell proliferation, comparable to the one obtained with a concentration 100 times smaller of PCA-4230. In the case of aspirin this result has been confirmed by other authors (J. Bernhardt, K. Rogalla, T.F. Luscher, et al., J. Immunogenet.m (1993), 21:973-976) that showed that the inhibition of the proliferation of smooth muscle cells is only achieved with aspirin concentrations higher than 270 µM.

### EXAMPLE 2

### Effect of PCA-4230 on altered cell communication in vascular human arteriosclerotic smooth muscle cells

Materials and Methods. Non-arteriosclerotic aorta smooth muscle cells (NASA), obtained from biopsies of organs to be used in heart transplants were used. The atheromatous smooth muscle cells (ASA) were obtained from biopsies obtained from arteriosclerotic arteries coming from aorta reconstruction. These biopsies were used as a source for cultures by explant, avoiding the use of enzymatic additives or detergents. The cells were left to grow until confluence, in M199 medium, supplemented with 5% BFS and antibiotics as in the previous example. The pureness of the cultures was determined by specific staining of the same with anti-α actin of smooth muscle cells.

Intracellular communication was studied by evaluating the transference of the fluorescent dye Lucifer Yellow from a single cell, in which it is inserted by microinjection, to the surrounding cells. The extent of this transference of dye was determined in 40 normal cells and in 100 atheromatous cells.

The activity of PCA-4230 was studied by measuring the transference of dye in atheromatous cell cultures before and after incubation with 200ng/ml of PCA-4230 (final concentration 0.4 µM). After a first evaluation of the transference of dye by atheromatous cells, the cells were incubated for 1 hour with PCA-4230 0.4 µM and the transference of a fresh injection of Lucifer Yellow was re-evaluated. The same experiment was carried out with Verapamyl 0.4 µM, used as a standard drug with calcium antagonist activity and with 0.02% dimethylsulfoxide, the vehicle used in both cases.

To control the migration rate and the stages of cell division 15 individual cells, placed radially at the same distance from the original explant, were injected with the dye before and after incubation with PCA-4230. The same study was repeated with the vehicle and with Verapamyl.

Subsequently, the cells were fixed with glutaraldehyde and were studied by FFEM "Freeze Fracture Electron Microscopy" to determine the morphology of the gap junctions. The cells were frozen at -110^{º} C and were fractured. The breakage planes are the planes with less resistance that correspond to the surface of the inside membrane, between lipid bilayers.

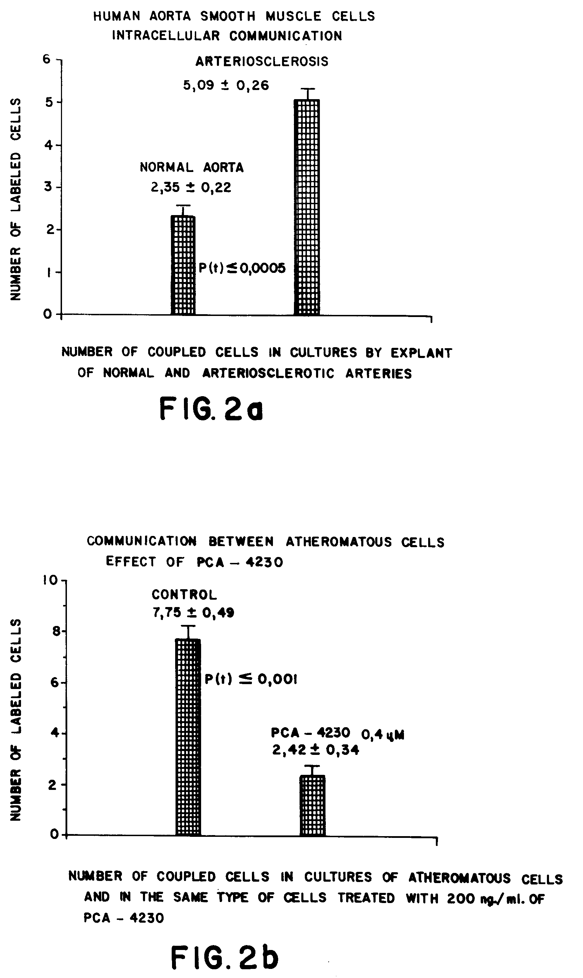

Results. The results of these experiments are expressed in Table II and in figures 2a and 2b. The smooth muscle cells coming from the non-arteriosclerotic artery cultures showed a frequency of 2.35 ± 0.22 cells labeled by injection, while those coming from atheromatous plaques , had a frequency of 7.75 ± 0.49 [P(t) ≤ 0.0005)]. After incubation with PCA-4230, there was a significant reduction of the number of cells labeled with the dye (2.42 ± 0.34 cells labeled by injection, P(t) ≤ 0.001). This density is similar to that observed in health artery cell cultures (Figures 2a and 2b): On the contary, Verapamyl did not show any significant effects on the transference of dye among atheromatous cells.

No differences were observed in the morphology of the gap junctions nor in the distribution and number of the same in the intracellular membranes of the cells treated with PCA-4230.

### Conclusions

1. Intracellular transference of molecules inceases significantly in smooth muscle cells derived from arteriosclerotic plaques compared with those derived from normal arterial tissue.
2. PCA-4230 significantly reduces intracellular transference of dye in arteriosclerotic smooth muscle cells. The functional result obtained in the gap junctions of these cells is comparable with that shown by cells coming from healthy tissues.
3. Verapamyl did not have any effect on the intracellular communication process of atheromatous cells.

**TABLE II**

| CELL TYPES | TREATMENT | N° CELLS LABELED BY INJECTION | P(t) |
|---|---|---|---|
| NORMAL (HEALTHY) | VEHICLE | 2,35 ± 0,22 | -- |
| | PCA-4230 | 2,40 ± 0,18 | n.s. |
| | VERAPAMYL | 2,28, ± 0,30 | n.s. |
| ATHEROMATOUS | VEHICLE | 7,75 ± 0,49 | -- |
| | PCA-4230 | 2,42 ± 0,34 | <0,001 |
| | VERAPAMYL | 7,20 ± 0,54 | n.s. |

### EXAMPLE 3

### Pharmaceutical Composition

Pills with enteric coating to be administered orally, each one containing 200 mg of PCA-4230.

### Composition per pill

| Pill: | |
|---|---|
| PCA-4230 | 200.00 mg |
| Cornstarch | 32.00 mg |
| Ac-di-sol | 20.00 mg |
| Avicel PH-101 (Cellulose) | 90.00 mg |
| Polyvinylpyrrolidone (Povidone) | 5.00 mg |
| Talc | 10.00 mg |
| Magnesium stearate | 2.00 mg |
| Aerosil 200 | 2.00 mg |
| Lactose | 455.00 mg |

### Insulating coating:

| | |
|---|---|
| Pharmacoat 606 | 22.50 mg |
| Propylene glycol | 2.50 mg |

### Enteric coating:

| | |
|---|---|
| Hydroxypropyl methylcellulose (HP-55) | 50.71 mg |
| Titanium dioxide | 1.51 mg |
| Ethyl alcohol | 2.78 mg |

### General conclusions

The proliferation of smooth muscle cells represents the key event in the development of arteriosclerotic lesions and of re-stenosis (R. Ross, Nature (1993), 362:801-809). Different studies carried out on experimental animals and on humans indicate that, in the majority of the cases, the main component and cause of the atheromatous lesion is migration and proliferation of smooth muscle cells from the media to the intima (R. Ross, Nature (1993), 362:801-809); A.M.C. Zanellato, A.C. Borrinone et al., Arteriosclerosis (1990), 10:996-1009); T. Tsukuda, M. Rosenfeld et al. Arteriosclerosis (1966), 6:601-613).

The specific cellular effects shown by PCA-4230 widely support the anti-ateroatherogenic activity of the compound.

It is obvious that the results included in the above examples are surprising, since no drug capable of inhibiting cell proliferation and bringing the behaviour of human atheromatous cells back to normal is known. These characteristics, together with a moderate hypolipidemic effect of PCA-4230 (M. Guivernau et al. Phase II Clinical Trial, Internal Report) and its already known anti-thrombotic activity make PCA-4230 a compound with exceptional characteristics. This fact is especially relevant, due to the fact that presently, there is no available drug capable of preventing cardiovascular disease, with inhibitory activity of the proliferation of smooth muscle cells, that brings the activity of atheromatous cells back to normal and that simultaneously has antiplatelet activity.

Most of the individuals defined as high cardiovascular risks have stenosis in arterial areas, high values of lipids in the blood and increased platelet activation, which gives PCA-4230 enormous clinical interest and relevant therapeutic implications.

A dosage range conceivable for treatment would be between 200-800 mg/day, depending on the patient and the specific response of the same to the medication. The exact daily dose will be determined by the doctor in each case.

## Claims

1. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate as a drug with effects on arteriosclerosis disease.

2. Application of 2-[N-(1,2-benzoisothiazolyl-3(2H) one-1,1-dioxide ethyl 5-ethoxycarbonyl-2,4,6-trimethyl-1,4-dihydropyridine-3-carboxylate as a drug with effects on ischemic and occlusive vascular disorders.

3. Application of 2-[N-(1,2-benzoisothiazolyl-3(2h) one-1,1-dioxide]ethyl 5-ethoxycarbonyl-2,4,6-trimethyl-1,4-dihydropyridine-3-carboxylate as a drug with inhibitory effects of cell proliferation.

4. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate as a drug with effects that bring cellular intercommunication back to normal.

5. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate, in accordance with claims 1-4 for use thereof in the treatment of patients with intermittent claudication.

6. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate, in accordance with claims 1-4 for use thereof in the treatment of arterial obstruction in patients with an altered lipid situation.

7. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate in the preparation of drugs to treat ateriosclerotic disease.

8. Application of 2-[N-(1,2-benziosothiazolyl-3(2H) one-1,1-dioxide]ethyl 5-ethoxycarbonyl-2,4,6-trimethyl-1,4-dihydropyridine-3-carboxylate in the preparation of drugs to treat ischemic and occlusive vascular disorders.

9. Application of 2-[N-(1,2-benzoisothiazolyl-3(2h) one-1,1-dioxide]ethyl 5-ethoxycarbonyl-2,4,6-trimethyl-1,4-dihydropyridine-3-carboxylate in the preparation of drugs to treat diseases that evidence pathologically high cell proliferation.

10. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate in the preparation of drugs to treat diseases that evidence pathologically high cell intercommunication.

11. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate in the preparation of drugs to treat intermittent claudication.

12. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate in the preparation of a pharmaceutical composition that contains this product as the active ingredient and a pharmaceutically acceptable vehicle or solvent, in accordance with any of the above claims.

13. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate , according to the above claims wherein said products are used alone or together with one or more agents with vascular activity such a β-blocking agents, calcium channel antagonists and other drugs with vasodilator activity, as well as hypolipidemic drugs.

14. Application of 2-[N-(1,2-benzoisothiazolyl-3(2H) one-1,1-dioxide]ethyl 5-ethoxycarbonyl-2,4,6-trimethyl-1,4-dihydropyridine-3-carboxylate in the manufacture of preparations to be administered orally or parenterally, in accordance with any of the above claims.
